# EUROPEAN PATENT SPECIFICATION

(11) **EP 4 601 595 B1**
(45) Date of publication and mention of the grant of the patent: **25.03.2026**
(21) Application number: 23793482.3
(22) Date of filing: 13.10.2023
(51) Int. Cl.: A41B 13/00, A61F 13/62

(54) **MECHANICAL FASTENER WITH HIGH NUMBER OF FASTENING ELEMENTS, FASTENING LAMINATE, AND ABSORBENT ARTICLE**
MECHANISCHES BEFESTIGUNGSELEMENT MIT HOHER ANZAHL VON BEFESTIGUNGSELEMENTEN, BEFESTIGUNGSLAMINAT UND SAUGFÄHIGER ARTIKEL
FIXATION MÉCANIQUE À NOMBRE ÉLEVÉ D'ÉLÉMENTS DE FIXATION, STRATIFIÉ DE FIXATION ET ARTICLE ABSORBANT

(30) Priority: 14.10.2022 US 202263416331 P
(43) Date of publication of application: 20.08.2025
(73) Proprietor: 3M Innovative Properties Company, Saint Paul, Minnesota 55133-3427 (US)
(72) Inventor: MORISHITA, Kenichiro, Tokyo 141-8684 (JP); GORMAN, Michael R., Saint Paul, Minnesota 55133-3427 (US); NAGATA, Hiroyasu, Tokyo 141-8684 (JP); NIEMI, Scott M., Saint Paul, Minnesota 55133-3427 (US)
(74) Representative: Bergen, Katja
(86) International application number: PCT/IB2023/060367
(87) International publication number: WO 2024/079720

(56) References cited:
- US-A1- 2012 151 722
- US-A1- 2014 350 507

## Description

### CROSS-REFERENCE TO RELATED APPLICATIONS

This application claims priority to U.S. Provisional Application No. 63/416,331 filed October 14, 2022.

### BACKGROUND

Mechanical fasteners, which are also called hook and loop fasteners, typically include a plurality of closely spaced upstanding projections with loop-engaging heads useful as hook members, and loop members typically include a plurality of woven, nonwoven, or knitted loops. Mechanical fasteners are useful for providing releasable attachment in numerous applications. For example, mechanical fasteners are widely used in wearable disposable absorbent articles to fasten such articles around the body of a person. In typical configurations, a hook strip or patch on a fastening tab attached to the rear waist portion of a diaper or incontinence garment, for example, can fasten to a landing zone of loop material on the front waist region, or the hook strip or patch can fasten to the backsheet (e.g., nonwoven backsheet) of the diaper or incontinence garment in the front waist region. Mechanical fasteners are also useful for disposable articles such as sanitary napkins. A sanitary napkin typically includes a back sheet that is intended to be placed adjacent to the wearer's undergarment. The back sheet may comprise hook fastener elements to securely attach the sanitary napkin to the undergarment, which mechanically engages with the hook fastener elements.

Hook and loop fastening systems can include at least two engagement strength characteristics: peel strength and shear strength. Peel strength corresponds to the force required to disengage the fastening members from one another by peeling one fastening member upward and away from the other fastening member. Shear strength corresponds to the force required to disengage the fastening members from one another by pulling at least one of the fastening members away from the other in a plane that is parallel to the fastening members. Typically, the engagement strength of the fastening members is higher in shear than in peel. While the peel strength may be a factor when the fastening members are intentionally separated, typically, the shear strength is responsible for holding the fastening members together during normal use.

U.S. Pat. Nos. 5,679,302 (Miller et al.), 10,973,710 (Peltier et al.), and 10,165,833 (Pariseau et al.) describe mechanical fasteners in which the mechanical fastening elements are present at a density of up to 1550 per square centimeter. U.S. Pat. No. 8,845,943 (Hertlein et al.) describes a method of making a structured surface such as a mechanical fastener. U.S. Pat. Nos. 5,392,498 (Goulait et al.), 7,162,780 (Martin et al.), and 7,578,812 (Datta et al.) describe skin-friendly hook fasteners. US2014/350507 also discloses fastening systems of the prior art.

### SUMMARY

The present disclosure provides a mechanical fastener with a relatively high pin density and a relatively high cap area percentage and also provides fastening laminates and absorbent articles that contain the mechanical fastener. The mechanical fasteners of the present disclosure have an unexpectedly soft tactile feel, and, in some embodiments, it is difficult for one to distinguish the feel of the side of the fastener having mechanical fastening elements and the side of the fastener having no fastening elements. Despite its relatively high pin density and a relatively high cap area percentage, the mechanical fastener disclosed herein has comparable or better engagement to loop materials than typical mechanical fasteners as measured by shear and peel testing.

In one aspect, the present disclosure provides a mechanical fastener including a thermoplastic backing and multiple upstanding fastening elements having posts with proximal ends integrally formed with the thermoplastic backing and distal ends comprising spaced-apart caps larger in cross-section area than a cross-sectional area of the posts. The multiple upstanding fastening elements are present in a density in a range from 1000 per square centimeter to 5000 per square centimeter, and a sum of the cross-sectional area of the spaced-apart caps is in a range from 20 to 70 percent of an area of the mechanical fastener. The spaced-apart caps are circular, oval, or regular polygonal having at least five sides and do not have a dimension greater than 300 micrometers.

In another aspect, the present disclosure provides a laminate including the mechanical fastener described above attached to a carrier.

In another aspect, the present disclosure provides an absorbent article that includes the mechanical fastener or the laminate described above.

In this application, terms such as "a", "an" and "the" are not intended to refer to only a singular entity but include the general class of which a specific example may be used for illustration. The terms "a", "an", and "the" are used interchangeably with the term "at least one". The phrases "at least one of" and "comprises at least one of" followed by a list refers to any one of the items in the list and any combination of two or more items in the list. All numerical ranges are inclusive of their endpoints and non-integral values between the endpoints unless otherwise stated.

The term "upstanding" refers to posts that protrude from the thermoplastic backing and includes posts that stand perpendicular to the backing and posts that are at an angle to the backing other than 90 degrees.

The term "spaced-apart" refers to caps that are formed to have a distance between them. "Spaced-apart" caps do not touch each other when the thermoplastic backing is in an unbent configuration.

The term "multiple" means more than one and can include any desired number of upstanding fastening elements.

The terms "first" and "second" are used in this disclosure in their relative sense only. It will be understood that, unless otherwise noted, those terms are used merely as a matter of convenience in the description of one or more of the embodiments. As used herein, the "first" surface of thermoplastic backing bears the upstanding fastening elements, and the "second" surface of the thermoplastic backing is opposite the first surface.

The above summary of the present disclosure is not intended to describe each disclosed embodiment or every implementation of the present disclosure. The description that follows more particularly exemplifies illustrative embodiments. It is to be understood, therefore, that the drawings and following description are for illustration purposes only and should not be read in a manner that would unduly limit the scope of this disclosure.

### BRIEF DESCRIPTION OF THE DRAWINGS

The disclosure may be more completely understood in consideration of the following detailed description of various embodiments of the disclosure in connection with the accompanying drawings, in which:
FIG. 1 is a side view of an exemplary upstanding fastening element on a thermoplastic backing useful in the mechanical fastener disclosed herein, wherein the various dimensions of the upstanding fastening element are shown; and
FIG. 2 is a perspective view of an exemplary disposable absorbent article that includes a mechanical fastener according to the present disclosure.

### DETAILED DESCRIPTION

Reference will now be made in detail to embodiments of the disclosure, one or more examples of which are illustrated in the drawings. Features illustrated or described as part of one embodiment can be used with other embodiments to yield still a third embodiment. It is intended that the present disclosure include these and other modifications and variations.

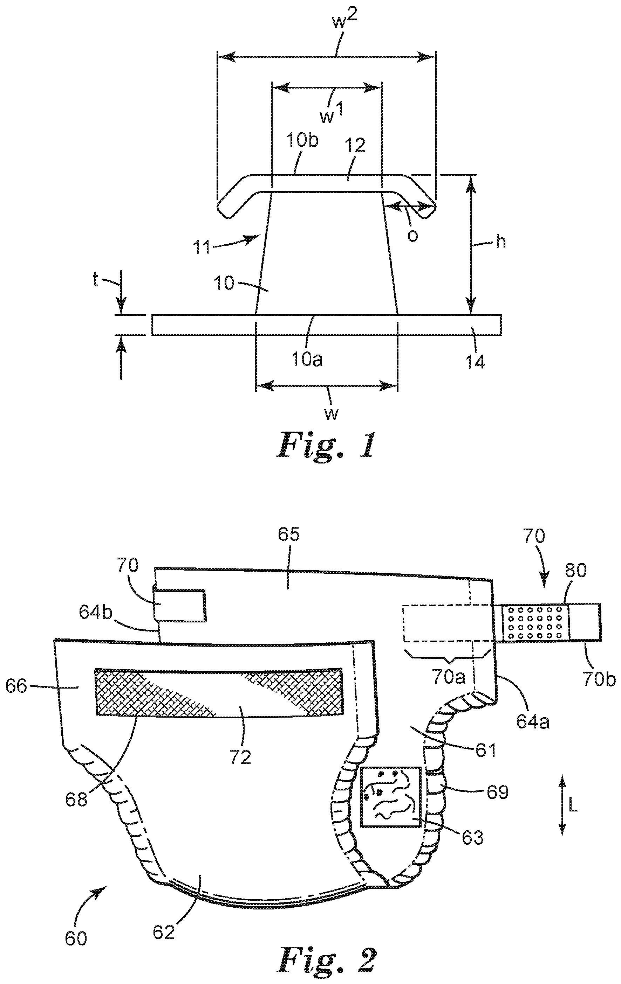

Referring to FIG. 1, the mechanical fastener according to the present disclosure includes a thermoplastic backing 14 with multiple upstanding fastening elements 11 attached to the thermoplastic backing 14. As shown in the embodiment illustrated in FIG. 1, the multiple upstanding fastening elements 11 have posts 10 with proximal ends 10a attached to and integrally formed with the thermoplastic backing 14 and distal ends 10b comprising spaced-apart caps 12 larger in cross-section area than a cross-sectional area of the post 10. Cap 12 extends an overhang distance "o" beyond the post. The height "h" of the upstanding fastening element is the distance between the thermoplastic backing 14 and the distal end 10b of the upstanding fastening element 11 as shown in FIG. 1. The thickness "t" of the thermoplastic backing 14, the width "w" of the post at its base, the width "w¹" of the post just below the cap, and the width "w²" of the cap are also shown in FIG. 1. For the purposes of this application, all dimensions of the upstanding fastening elements described herein with reference to FIG. 1 and in the claims are measured by optical microscopy.

In the mechanical fastener of the present disclosure, the upstanding fastening elements have a density in a range from 1000 per square centimeter (cm²) (6450 per square inch, in²) to 5000/cm² (32,250/ in²), 4000/cm² (25,800/in²), or 3000/cm² (19,350/in²). In some embodiments, the density of the upstanding fastening elements is in a range from 1000/cm² (6450/in²) to 1400/cm² (9030/in²), from 1050/cm² (6770/in²) to 1400/cm² (9030/in²), from 1050/cm² (6770/in²) to 5000/ cm² (32,250/in²), from 1600/cm² (10,320/in²) to 5000/ cm² (32,250/in²), from 1600/cm² (10,320/in²) to 4000/ cm² (25,800/in²), from 1600/cm² (10,320/in²) to 3000/ cm² (19,350/in²), from 1500/cm² (9675/in²) to 4000/ cm² (25,800/in²), from 1650/cm² (10,642/in²) to 4000/ cm² (25,800/in²), or from 1650/cm² (10,642/in²) to 3000/ cm² (19,350/in²). In some embodiments, the density of the upstanding fastening elements is about 1240/cm² (8000/in²). In some embodiments, the density of the upstanding fastening elements is about 2480/cm² (16,000/in²).

In some embodiments, the distance between the posts (i.e., pin spacing) is approximately equal in both directions. That is, in such embodiments, the posts 10 of the upstanding fastening elements are substantially evenly spaced. "Substantially evenly spaced" means that the distances between posts 10 differ from each other by up to 10, 7.5, or 5 percent. A variety of configuration of upstanding fastening elements may be useful, for example, square arrays, staggered arrays, and sinusoidal arrays.

Referring again to FIG. 1, the spaced-apart caps 12 have a maximum width dimension "w²" of up to 300 micrometers (µm). With the width dimension "w²" is greater than 300 µm, the cap may bend when the mechanical fastener is exposed to a peel force, decreasing the strength of engagement. Moreover, the width dimension of the caps should allow them to be spaced apart. If the caps are touching, as in U.S. Pat. No. 8,845,943 (Hertlein et al.), the upstanding elements are unable to engage loops and cannot function as mechanical fastening elements. The width dimension "w²" is measured by viewing the caps from above. The term "width dimension" should be understood to be the diameter of a cap 12 with a circular cross-section. When the cap 12 has more than one width dimension (e.g., in a cap with an oval cross-sectional shape), "w²" is measured at the widest point of the cap 12 (that is, the longer axis of the oval). In some embodiments, the spaced-apart caps 12 have a width dimension "w²" in a range from 150 µm to 300 µm or 150 µm to 270 µm. In some embodiments, the spaced-apart caps 12 have a width dimension "w²" in a range from 80 µm to 245 µm or 80 µm to 225 µm. In some embodiments, the density of the multiple upstanding fastening elements is in a range from 1000/cm² to 1400/cm², and the spaced-apart caps have a dimension in a range from 150 µm to 300 µm or 150 µm to 270 µm. In some embodiments, the density of the multiple upstanding fastening elements is in a range from 1500/cm² to 4000/cm², and wherein the spaced-apart caps have a dimension in a range from 80 micrometers to 225 micrometers.

The density of the upstanding fastening elements and the cap area may be used to determine a total cap area in the mechanical fastener disclosed herein. The sum of the cross-sectional area of the caps is in a range from 20% to 70% of the area of the mechanical fastener. This percentage can be calculated from the total cap area divided by the overall area of the mechanical fastener. In some embodiments, the sum of the cross-sectional area of the caps is in a range from 25% to 70%, 20% to 65%, 25% to 60%, or from 31% to 60% of the area of the mechanical fastener.

The upstanding fastening elements in the mechanical fastener of the present disclosure may have a variety of shapes. The fastening element may be in the shape of a mushroom or a nail (e.g., with a circular, oval, or regular polygonal head enlarged with respect to the post). Upstanding fastening element having these shapes are typically considered multi-directional. In some of these embodiments, the cap extends beyond the posts on at least two opposing sides of the post. Thus, the cap has an overhang as described below. The extent of overhang on the two opposing sides may or may not be equal. In some of these embodiments, the fastening element is in the shape of a mushroom (e.g., with a circular or oval head enlarged with respect to the post). In some embodiments, the caps may extend beyond the posts in all directions although the amount of overhang may not be equal in all directions. In some embodiments, at least a portion of the spaced-apart caps is deflected toward the thermoplastic backing. This can be carried out after the caps are formed, for example, using a method described below.

The spaced-apart caps in the mechanical fastener of the present disclosure have a cross-sectional shape that is circular, oval, or regular polygonal having at least five sides. The regular polygonal shape may have five, six, eight, ten, or twelve sides, for example. In some embodiments, the regular polygonal shape is regular hexagonal. A polygon is regular when all angles are equal and all sides are equal. In the regular polygonal shaped caps, the angles may be slightly rounded or radiused, but the shape can still be recognized as having at least 5 (in some embodiments, 6) sides. In some embodiments, the caps have a circular (or generally circular) cross-sectional shape. Generally circular can mean that the shape has some deviation from circular that would result from a manufacturing process, as would be understood by a person skilled in the art.

The spaced-apart caps may have a grooved surface or a smooth surface. In some embodiments, the spaced-apart caps have a smooth surface. In embodiments in which the spaced-apart caps have a grooved surface, it may be useful for the sum of the cross-sectional area of the spaced-apart caps to be in a range from 20% to 40% of the area of the mechanical fastener, for example, for a softer the tactile feel as shown in the Examples, below.

The mechanical fastener of the present disclosure, with the densities and cap areas described above in any of their embodiments, have an unexpectedly soft feeling when the upstanding fastening elements come into contact with a person's skin. In some embodiments, it is difficult for one to distinguish the feel of the side of the fastener having mechanical fastening elements and the side of the fastener having no fastening elements.

The thermoplastic backing in the mechanical fastener of the present disclosure can have a variety of thicknesses. In some embodiments, the thickness of the thermoplastic backing "t" in the mechanical fastener disclosed herein is in a range from 20 µm to 200 µm. In some embodiments, the thickness of the thermoplastic backing "t" is in a range from 30 µm to 100 µm, 30 µm to 90 µm, 30 µm to 80 µm, 25 µm to 80 µm, or 35 µm to 60 µm.

The mechanical fastener of the present disclosure typically feels soft and flexible when it is bent. For the purposes of this disclosure, bending softness is measured using a KES-FB2-S Pure Bending Tester from Kato Tech Co., Ltd., Kyoto, Japan, using the method described in the Examples, below. In some embodiments, the bending softness of the mechanical fastener is at least 0.01 (grams of force (gf) times centimeter(cm))/cm (gf*cm/cm) and not more than 0.1 gf*cm/cm, 0.09 gf*cm/cm, 0.08 gf*cm/cm, 0.07 gf*cm/cm, 0.06 gf*cm/cm, or 0.05 gf*cm/cm.

In some embodiments of the mechanical fastener according to the present disclosure, the upstanding fastening elements 11 have a maximum height "h" of up to 350 µm, in some embodiments up to 325 µm or 300 µm. In some embodiments, the mechanical fastener according to the present disclosure has upstanding fastening elements 11 with a minimum height "h" of 80 µm, in some embodiments, 100 µm, 140 µm, or 150 µm. In some embodiments, the mechanical fastener according to the present disclosure has upstanding fastening elements 11 with a height "h" in a range from 150 µm to 300 µm, 80 µm to 350 µm, 90 µm to 335 µm, 100 µm to 325 µm, 100 µm to 300 µm, or 155 µm to 250 µm.

Typically, the upstanding fastening elements 11 in the mechanical fastener according to the present disclosure have aspect ratio (that is, a ratio of height "h" over the widest width dimension "w") of up to about 2:1, 1.5:1, or 1.2:1. The posts 10 may have a cross-section with a maximum width dimension "w" of up to 250 µm and at least 75 µm. In some embodiments, the posts 10 have a cross-section with a width dimension "w" in a range from 75 µm to 200 µm or 85 µm to 190 µm. The term "width dimension" should be understood to include the diameter of a post 10 with a circular cross-section. When the post 10 has more than one width dimension (e.g., in a post with an oval cross-sectional shape), the width "w" refers to the widest width dimension, and the aspect ratio described herein is the height over the widest width dimension.

In the mechanical fastener according to the present disclosure, the upstanding fastening elements 11, which may be made, for example, by any of the methods described below, may have a post 10 that tapers, for example, from the proximal end 10a toward the distal end 10b. The proximal end 10a may have a larger width dimension "w" than the post 10 adjacent the cap 12, where the width dimension is "w¹" shown in FIG. 1. In some embodiments, the posts 10 have a width dimension "w¹" immediately under the cap in a range from 60 µm to 200 µm, 60 µm to 175 µm, 65 µm to 175 µm, or 75 µm to 150 µm. The tapering may facilitate the removal of the post 10 from the mold surface in the methods described below.

The mechanical fastening elements described herein have spaced-apart caps that are larger in cross-sectional area than the cross-sectional area of the posts. In some embodiments, the cross-section area of the post is measured just below the cap (e.g., at the point where width "w¹" is shown). In some embodiments, a ratio of a width dimension of the cap to the post measured at the proximal end is typically at least 1.01:1 or 1.2:1 and may be up to 2:1. The width "w²" of the cap 12 and the width "w¹" of the post 10 toward the distal end 10b are related to the cap overhang "o". Specifically, the cap overhang "o" in the illustrated embodiment is calculated from the formula (cap width "w²" - post width "w¹" at the distal end) divided by 2. In some embodiments of the mechanical fasteners according to the present disclosure, the overhang distance is up to 90 µm. In other words, the overhang extends up to 90 µm beyond the post. In some embodiments, the overhang is up to 80 µm, 70 µm, 60 µm, or 50 µm. In some embodiments, the overhang is at least 5 µm or 10 µm. The overhang may be in the range, for example, from 5 µm to 90 µm, 10 µm to 80 µm, 10 µm to 60 µm, or 15 µm to 65 µm.

In some embodiments, the mechanical fastener has a basis weight of up to 100 grams per square meter (gsm), up to 95 gsm, or up to 90 gsm. It can be desirable to make a basis weight as low as possible, for example, to minimize the amount of material used to make the mechanical fastener. In some embodiments, the basis weight of the mechanical fastener is at least 25 gsm, at least 45 gsm, at least 50 gsm, at least 55 gsm, at least 60 gsm, at least 70 gsm, more than 75 gsm, or at least 76 gsm. In some embodiments, the mechanical fastener has a basis weight in a range from 45 gsm to 95 gsm or from 55 gsm to 95 gsm. In some embodiments, the mechanical fastener has a basis weight in a range from 76 gsm to 95 gsm. Various factors influence the basis weight of a mechanical fastener. Since the mechanical fastener of the present disclosure has a high density of upstanding fastening elements, it can be useful to select fastening elements with lower widths (e.g., w and w¹) and lower heights and a thermoplastic backing with a lower thickness to lower the basis weight of the mechanical fastener.

Many thermoplastic materials are useful for mechanical fasteners according to the present disclosure. Suitable thermoplastic materials for the thermoplastic backing with upstanding fastening elements include polyolefin homopolymers such as polyethylene and polypropylene, copolymers of ethylene, propylene and/or butylene; copolymers containing ethylene such as ethylene vinyl acetate and ethylene acrylic acid; polyesters such as poly(ethylene terephthalate), polyethylene butyrate and polyethylene naphthalate; polyamides such as poly(hexamethylene adipamide); polyurethanes; polycarbonates; poly(vinyl alcohol); ketones such as polyetheretherketone; polyphenylene sulfide; poly(acrylonitrile-butadiene-styrene); plasticized polyvinylchlorides; and mixtures thereof. Typically, the thermoplastic is a polyolefin (e.g., polyethylene, polypropylene, polybutylene, ethylene copolymers, propylene copolymers, butylene copolymers, and copolymers and blends of these materials). In some embodiments, the thermoplastic comprises polypropylene. In some embodiments, the thermoplastic has a bulk flexural modulus of at least 300 megaPascals (MPa) or at least 500 or 1000 MPa. In some embodiments, the thermoplastic has a bulk flexural modulus of up to 3000 MPa or up to 2500 or 2000 MPa. The various thermoplastic materials described above can be formulated into a master batch having a desired property (e.g., color); however, the presence or absence of dyes, pigments, or other colorants are not essential to this disclosure.

In some embodiments, the thermoplastic backing with upstanding fastening elements can be made from a multilayer or multi-component melt stream of thermoplastic materials. This can result in fastening elements formed at least partially from a different thermoplastic material than the one predominately forming the backing. Various configurations of upstanding posts made from a multilayer melt stream are shown in U. S. Pat. No. 6,106,922 (Cejka et al.), for example. A multilayer or multi-component melt stream can be formed by any conventional method. A multilayer melt stream can be formed by a multilayer feedblock, such as that shown in U.S. Pat. No. 4,839,131 (Cloeren). A multicomponent melt stream having domains or regions with different components could also be used. Useful multicomponent melt streams could be formed by use of inclusion co-extrusion die or other known methods (e.g., that shown in U.S. Pat. No. 6,767,492 (Norquist et al.).

In the mechanical fasteners according to the present disclosure, the thermoplastic backing and the upstanding posts are integral (that is, formed at the same time as a unit, unitary). In some embodiments, the thermoplastic backing and the upstanding fastening elements are made from the same thermoplastic material. The thermoplastic backing is typically in the form of a sheet or web that may have an essentially uniform thickness with the upstanding fastening elements directly attached to the thermoplastic backing. Upstanding posts on a backing can be made, for example, by conventional extrusion through a die and cast molding techniques. In some embodiments, a thermoplastic material is fed onto a continuously moving mold surface with cavities having the inverse shape of the upstanding posts. The height of the posts is determined by the depth of the cavities. The thermoplastic material can be passed between a nip formed by two rolls or a nip between a die face and roll surface, with at least one of the rolls having the cavities (i.e., at least one of the rolls is a tool roll). Pressure provided by the nip forces the resin into the cavities. In some embodiments, a vacuum can be used to evacuate the cavities for easier filling of the cavities. The nip has a gap that is typically sufficiently big such that a coherent backing is formed over the cavities. The mold surface and cavities can optionally be air or water cooled before stripping the integrally formed backing and upstanding posts from the mold surface such as by a stripper roll.

Suitable tool rolls can be made, for example, by forming (e.g., by computer numerical control with drilling, photo etching, using galvanic printed sleeves, laser drilling, electron beam drilling, metal punching, direct machining, or lost wax processing) a series of holes having the inverse shape of the upstanding posts into the cylindrical face of a metal mold or sleeve. Other suitable tool rolls include those formed from a series of plates defining a plurality of post-forming cavities about its periphery such as those described, for example, in U.S. Pat. No. 4,775,310 (Fischer). Cavities may be formed in the plates by drilling or photoresist technology, for example. Still other suitable tool rolls may include wire-wrapped rolls, which are disclosed along with their method of manufacturing, for example, in U.S. Pat. No. 6,190,594 (Gorman et al.). The exposed surface of the mold, sleeve, plate, or wire may be coated to impart surface properties such as increased wear resistance, controlled release characteristics, and controlled surface roughness. The coating, if present, can be selected so that the adhesion of the thermoplastic material to the tool roll is less than the cohesion of the thermoplastic material at the time of the removal of the thermoplastic backing from the tool roll.

Another method for forming a thermoplastic backing with upstanding posts includes using a flexible mold belt defining an array of upstanding post-shaped cavities as described in U.S. Pat. No. 7,214,334 (Jens et al.). The mold belt is trained about first and second rolls, and a source of molten thermoplastic material is arranged to deliver the thermoplastic to the mold belt. The apparatus is constructed to force the plastic resin into the upstanding post-shaped cavities of the belt under pressure in a gap to mold the array of upstanding posts while forming the thermoplastic web layer.

Useful upstanding posts on a backing may have a variety of heights above the backing, which may be changed, for example, after a capping process described below to provide the upstanding fastening elements of the mechanical fastener described herein. For example, the upstanding posts can have a maximum height (above the backing) of up to 400 micrometers (µm), in some embodiments up to 350 µm or 300 µm. The upstanding posts can have a minimum height of at least 150 µm, in some embodiments, at least 160 µm, 175 µm, or 200 µm. Useful upstanding posts before capping can have a height in a range from 150 µm to 400 µm or 150 µm to 350 µm.

Posts formed upon exiting the cavities typically do not have caps, but they can be subsequently formed into upstanding fastening elements described herein by a capping method as described in No. 5,607,635 (Melbye et al.). Typically, the capping method includes deforming the tip portions of the upstanding posts using heat and/or pressure. The heat and pressure, if both are used, could be applied sequentially or simultaneously. In some embodiments, deforming comprises contacting the distal tips of the upstanding posts with a heated surface. The heated surface may be a flat surface or a textured surface such as that disclosed in U.S. Pat. Nos. 6,708,378 (Parellada et al.) or 5,868,987 (Kampfer et al.). In some embodiments, wherein the thermoplastic backing with upstanding fastening elements is a web of indefinite length, deforming the distal tips of the posts to form caps includes moving the web in a first direction through a nip having a heated surface member and an opposing surface member such that the heated surface member contacts the distal tips. In these embodiments, the heated surface may be, for example, a capping roll. In some embodiments, the surfaces used to contact the distal tips may not be heated. In these embodiments, the deformation is carried out with pressure and without heating. In some embodiments, the heated surface may be a heated roll opposite a curved support surface forming a variable nip having a variable nip length as described, for example, in U. S. Pat. No. 6,368,097 (Miller et al.). The curved support surface may curve in the direction of the heated roll, and the heated roll may include a feeding mechanism for feeding the thermoplastic backing with upstanding posts through the variable nip to compressively engage the web between the heated roll and the support surface. In some embodiments, heating is carried out below a melt temperature of the distal tips. When the thermoplastic material used to form the upstanding posts is a copolymer (e.g., copolymers of ethylene and propylene), the distal tips may have more than one melt temperature. In these embodiments, "below a melt temperature of the distal tips" means below at least one of the melt temperatures.

In some embodiments, distal caps of the upstanding fastening elements are reshaped after they are formed. For example, passing a thermoplastic backing having upstanding capped posts through a gapped nip of a heated rubber roll and a backup roll causes the overhanging portions of the distal cap, that extend beyond the post, to be pushed down toward the backing. This process is described in U.S. Pat. No. 6,132,660 (Kampfer).

**In** addition to the continuous methods described above, it is also envisioned that thermoplastic backings having upstanding fastening elements can be prepared using batch processes (e.g., single piece injection molding). The thermoplastic backing may have any suitable dimension, but length (L) and width (W) dimensions of at least 10 centimeters may be useful.

Another method for forming a thermoplastic backing with upstanding fastening elements is profile extrusion, which is described, for example, in U.S. Pat. No. 4,894,060 (Nestegard). Typically, in this method a thermoplastic flow stream is passed through a patterned die lip (e.g., cut by electron discharge machining) to form a web having downweb ridges. The ridges can then be transversely sliced at spaced locations along the extension of the ridges to form upstanding fastening elements with a small separation caused by the cutting blade. The separation between upstanding fastening elements is then increased by stretching. However, it should be understood that "upstanding fastening elements" do not include such ridges before they are cut. Such ridges themselves would not be considered to have a "loop-engaging overhang" because they would not be able to engage loops before they are cut and stretched. In some embodiments, the mechanical fasteners according to the present disclosure are not made by profile extrusion.

In the mechanical fastener according to the present disclosure, the upstanding fastening elements, which may be made, for example, by any of the methods described above, may have a variety of cross-sectional shapes. For example, the cross-sectional shape of the post may be a polygon (e.g., square, rectangle, hexagon, or pentagon), which may be a regular polygon or not, or the cross-sectional shape of the post may be curved (e.g., circular or elliptical). In some embodiments, the cross-sectional shape of the post is a regular polygon having at least five sides. The regular polygonal shape may have five, six, eight, ten, or twelve sides, for example. In some embodiments, the regular polygonal shape is regular hexagonal. In the regular polygonal shaped posts, the angles may be slightly rounded or radiused. In some embodiments, the posts taper and decrease in size from the proximal ends toward the distal ends.

The overhang in the upstanding fastening elements disclosed herein is typically considered to be loop-engaging. The term "loop-engaging" as used herein relates to the ability of an upstanding fastening element to be mechanically attached to a loop material. The loop-engageability of hook elements may be determined and defined by using standard woven, nonwoven, or knit materials. A region of posts with loop-engaging overhangs the distal ends generally will provide, in combination with a loop material, at least one of a higher peel strength, higher dynamic shear strength, or higher dynamic friction than a region of posts without loop-engaging overhangs.

The thermoplastic backing may have an essentially uniform cross-section, or the thermoplastic backing may have additional structure beyond what is provided by the upstanding fastening elements, which may be imparted, for example, by at least one of the forming rolls described above.

In some embodiments, the thickness of the thermoplastic backing as described above in any of its embodiments is achieved for the mechanical fastener disclosed herein by the gap of the nip between two rolls or the die face and a roll in the extrusion and cast molding process described above. An endless metal or polymer belt or a metal belt coated with polymer may be useful to provide even pressure in the nip. In some embodiments, the thickness of the thermoplastic backing is not altered by stretching. In some embodiments, the thermoplastic backing does not have stretch-induced molecular orientation. Whether a thermoplastic backing has stretch-induced molecular orientation can be determined by standard spectrographic analysis of the birefringent properties of the oriented thermoplastic polymer forming the backing. The mechanical fastener having stretch-induced molecular orientation may also be said to be birefringent, which means that the thermoplastic backing has different effective indexes of refraction in different directions.

In some embodiments, the thermoplastic backing has stretch-induced molecular orientation. In some of these embodiments, the thermoplastic backing is stretched to a ratio in a range from 1.1 to 4 in at least one direction (e.g., the machine direction). When the thermoplastic backing is a web of indefinite length, for example, monoaxial stretching in the machine direction can be performed by propelling the thermoplastic web over rolls of increasing speed. The most versatile stretching method that allows for monoaxial, sequential biaxial, and simultaneous biaxial stretching of a thermoplastic web employs a flat film tenter apparatus. Such an apparatus grasps the thermoplastic web using a plurality of clips, grippers, or other film edge-grasping means along opposing edges of the thermoplastic web in such a way that monoaxial, sequential biaxial, or simultaneous biaxial stretching in the desired direction is obtained by propelling the grasping means at varying speeds along divergent rails. Increasing clip speed in the machine direction generally results in machine-direction stretching. Means such as diverging rails generally results in cross-direction stretching. Monoaxial and biaxial stretching can be accomplished, for example, by the methods and apparatus disclosed in U.S. Pat. No. 7,897,078 (Petersen et al.) and the references cited therein. Flat film tenter stretching apparatuses are commercially available, for example, from Brückner Maschinenbau GmbH, Siegsdorf, Germany.

In the present application, whether the thermoplastic backing has stretch-induced molecular orientation is measured with a retardance imaging system available from Lot-Oriel GmbH & Co., Darmstadt, Germany, under the trade designation "LC-PolScope" on a microscope available from Leica Microsystems GmbH, Wetzlar, Germany, under the trade designation "DMRXE" and a digital CCD color camera available from QImaging, Surrey, BC, Canada, under the trade designation "RETIGA EXi FAST 1394". The microscope is equipped with a 546.5 nm interference filter obtained from Cambridge Research & Instrumentation, Inc., Hopkinton, Mass., and 10x/0.25 objective.

For any of the embodiments of the mechanical fastener according to the present disclosure, the thermoplastic backing may be in the form of a roll, from which mechanical fastener patches, for example, may be cut in a size appropriate to the desired application. In this application, the thermoplastic backing may also be a patch that has been cut to a desired size. In some of these embodiments, the second surface of the thermoplastic backing (i.e., the surface opposite the first surface from which the upstanding fastening elements project) may be coated with an adhesive (e.g., a pressure sensitive adhesive). In such embodiments, when the thermoplastic backing is in the form of a roll, a release liner may be applied to the exposed adhesive.

In some embodiments of the mechanical fastener disclosed herein, the thermoplastic backing is not joined to a carrier, at least when it is initially formed. In other embodiments, the second surface of the thermoplastic backing (i.e., the surface opposite the first surface from which the upstanding fastening elements project) is joined to a carrier. The thermoplastic backing may be joined to a carrier, for example, by lamination (e.g., extrusion lamination), adhesives (e.g., pressure sensitive adhesives), or other bonding methods (e.g., ultrasonic bonding, compression bonding, or surface bonding). The thermoplastic backing may also be joined to a carrier during the formation of the thermoplastic backing with upstanding posts. The resulting article may be a fastening laminate, for example, a fastening tab joined to the back sheet of an absorbent article useful for joining the front waist region and the rear waist region of an absorbent article.

The carrier, which in some embodiments may be joined to the second surface of the thermoplastic backing, may be continuous (i.e., without any through-penetrating holes) or discontinuous (e.g. comprising through-penetrating perforations or pores). The carrier may comprise a variety of suitable materials including woven webs, non-woven webs (e.g., spunbond webs, spunlaced webs, airlaid webs, meltblown web, and bonded carded webs), textiles, plastic films (e.g., single- or multilayered films, coextruded films, or films comprising foam layers), and combinations thereof. In some embodiments, the carrier is a fibrous material (e.g., a woven, nonwoven, or knit material). The term "nonwoven" when referring to a carrier or web means having a structure of individual fibers or threads which are interlaid, but not in an identifiable manner as in a knitted fabric. Nonwoven fabrics or webs can be formed from various processes such as meltblowing processes, spunbonding processes, spunlacing processes, and bonded carded web processes. In some embodiments, the carrier comprises multiple layers of nonwoven materials with, for example, at least one layer of a meltblown nonwoven and at least one layer of a spunbonded nonwoven, or any other suitable combination of nonwoven materials. For example, the carrier may be a spunbond-meltbond-spunbond, spunbond-spunbond, or spunbond-spunbond-spunbond multilayer material. Or, the carrier may be a composite web comprising a nonwoven layer and a dense film layer.

Fibrous materials that provide useful carriers may be made of natural fibers (e.g., wood or cotton fibers), synthetic fibers (e.g., thermoplastic fibers), or a combination of natural and synthetic fibers. Exemplary materials for forming thermoplastic fibers include polyolefins (e.g., polyethylene, polypropylene, polybutylene, ethylene copolymers, propylene copolymers, butylene copolymers, and copolymers and blends of these polymers), polyesters, and polyamides. The fibers may also be multi-component fibers, for example, having a core of one thermoplastic material and a sheath of another thermoplastic material.

One or more zones of the carrier may comprise one or more elastically extensible materials extending in at least one direction when a force is applied and returning to approximately their original dimension after the force is removed. The term "elastic" refers to any material that exhibits recovery from stretching or deformation. Likewise, "nonelastic" materials, which do not exhibit recovery from stretching or deformation, may be useful for the carrier as well.

Mechanical fasteners according to the present disclosure are useful components in fastening systems that include the mechanical fastener according to any of the above embodiments and a loop material. Mechanical fasteners of the present disclosure are useful with a wide variety of different loop materials. In some embodiments, the loop material is a low-loft loop material, which is desirable for use in fastening systems because of their low cost and low material-usage. Examples of low-loft materials include nonwoven materials, for example, made from any of the materials described above for carriers. In some embodiments, the loop material has a fiber basis weight in a range from 10 grams per square meter (gsm) to 50 gsm. Fiber basis weight is a basis weight of just the fiber in the loop material (e.g., removed from any backing).

The fastening laminate that can be formed after joining the thermoplastic backing to a carrier may be useful, for example, in absorbent articles. Some examples of absorbent articles have at least a front waist region, a rear waist region, and a longitudinal center line bisecting the front waist region and the rear waist region, wherein at least one of the front waist region or the rear waist region comprises the structured surface made according to the method disclosed herein. The fastening laminate may be in the form of a fastening tab that is bonded to at least one of the front waist region or the rear waist region extending outwardly from at least one of the left longitudinal edge or the right longitudinal edge of the absorbent article. In other embodiments, the fastening laminate may be an integral ear portion of the absorbent article.

FIG. 2 is a schematic perspective view of an embodiment of an absorbent article according to the present disclosure. The absorbent article is a diaper 60 having an essentially hourglass shape. The diaper comprises an absorbent core 63 between a liquid permeable top sheet 61 that contacts the wearer's skin and an outwardly facing liquid impermeable back sheet 62. Diaper 60 has a rear waist region 65 having two fastening tabs 70 arranged at the two longitudinal edges 64a, 64b of diaper 60. The diaper 60 may comprise an elastic material 69 along at least a portion of longitudinal side edges 64a and 64b to provide leg cuffs. The longitudinal direction "L" of the absorbent article (e.g., diaper 60) refers to the direction that the article extends from the front to rear of the user. Therefore, the longitudinal direction refers to the length of the absorbent article between the rear waist region 65 and the front waist region 66. The lateral direction of the absorbent article (e.g., diaper 60) refers to the direction that the article extends from the left side to the right side (or vice versa) of the user (i.e., from longitudinal edge 64a to longitudinal edge 64b in the embodiment of FIG. 2).

In FIG. 2, fastening tabs 70 are secured through their manufacturer's end 70a to the rear waist region 65. The user's end 70b of the fastening tab comprises a mechanical fastener 80 according to the present disclosure. In some embodiments, when attaching the diaper 60 to a wearer's body, the user's ends 70b of fastening tabs 70 can be attached to a target area 68 comprising fibrous material 72 which may be arranged on the back sheet 62 of the front waist region 66. Examples of loop tapes which may be applied to the target area 68 to provide an exposed fibrous material 72, are disclosed, for example, in U.S. Pat. No. 5,389,416 (Mody et al.), EP 0,341,993 (Gorman et al.), and EP 0,539,504 (Becker et al.). In other embodiments, the back sheet 62 comprises a woven or nonwoven fibrous layer which is capable of interacting with the user's ends 70b of the fastening tabs 70 comprising a mechanical fastener disclosed herein. Examples of such back sheets 62 are disclosed, for example, in U.S. Pat. Nos. 6,190,758 (Stopper) and 6,075,179 (McCormack et al.) and described in the Examples, below. Accordingly, in some embodiments, the disposable absorbent article disclosed herein is free of a target loop landing zone.

Although the embodiment illustrated in FIG. 2 is an absorbent article with attached fastening tabs, it is envisioned that the mechanical fastener disclosed herein would be equally useful in absorbent articles with larger areas of hooks. For example, the ears of the absorbent article can themselves comprise the mechanical fastener disclosed herein, or the absorbent article can have two target zones of loop material along the longitudinal edges of the back sheet in one waist region and two hook strips extending along the longitudinal edges of the absorbent article in the opposite waist region.

Fastening laminates according to the present disclosure may also be useful, for example, as fastening tabs for pants-style diapers such as those described in U.S. Pat. No. 5,531,732 (Wood). In some embodiments, the absorbent article according to the present disclosure is a disposable pants-style diaper having a fibrous outer covering or back sheet that can engage with upstanding fastening elements on a fastening tab disclosed herein. The fastening tab can be located on a seam or side panel portion of the pants-style diaper such that a free end of the fastening tab is able to engage the fibrous outer covering or back sheet. The fastening tab free end is useful, for example, for adjusting the pants-style diaper's circumferential fit or size (in other words, waist fit or size) by gathering the side panel portion. The side panel portion may be free of any integrally bonded absorbent core structure in some embodiments. In some embodiments, the pants-style diaper has at least one perforation line extending from the waist opening to one of the leg openings. The perforation is generally near the side seam, may be positioned toward the front portion of the diaper, and may be parallel or non-parallel to the side seam. In some embodiments, the pants-style diaper has a pair of perforation lines, one on each side of the diaper. The perforation line(s) can either be broken before the diaper is positioned around the user's torso or while the user is wearing the diaper. The fastening tab free end can then be used to refasten the diaper so that it is snug around the user's waist. The fastening tab free end may also be useful, for example, as a disposal means when the pants-style diaper is removed from the wearer (e.g., by tearing the side panel or perforation line). The fastening tab typically remains on the side panel portion. The diaper can then be rolled into a compact form for disposal, and the fastening tab can be used to keep the diaper in a rolled form.

The fastening laminate including the mechanical fastener disclosed herein may also be useful, for example, for absorbent articles such as sanitary napkins. A sanitary napkin typically includes a back sheet that is intended to be placed adjacent to the wearer's undergarment. The back sheet may comprise a thermoplastic backing with spaced-apart, upstanding capped posts to securely attach the sanitary napkin to the undergarment, which mechanically engages with the capped posts. The back sheet may formed with upstanding capped posts. In other embodiments, the mechanical fastener may be in the form of a strip or patch that is attached to the back sheet with adhesive or using another bonding mechanism.

According to U.S. Pat. No. 7,162,780 (Martin et al.), skin-friendly hook components should have an aggregate hook head area divided by the overall area of the hook component of 40% to 55% unless other factors such as softer resins or backings with variable thicknesses are used to make the hook more skin friendly. Martin et al. states that a hook density of 1000 to 2000 hooks per square inch (155 to 310 hooks per square centimeter), 1200 to 1800 hooks per square inch (186 to 279 hooks per square centimeter), or 1300 to 1600 hooks per square inch (202 to 248 hooks per square centimeter) provides optimum spacing for a skin-friendly and useful hook component for many garment applications. From this teaching, the skilled person would expect that a higher hook density with a large cap area would be less useful because there would be less room between the hooks to allow engagement with loops. Further, as explained in 8,845,943 (Hertlein et al.), when at least some caps touch at least one adjacent cap, there is not enough space around the caps for mechanical fastening engagement in multiple directions.

Unexpectedly, as shown in the Examples below, the mechanical fastener according to some embodiments of the present disclosure has improved softness relative to mechanical fasteners having a density of mechanical fastening elements of 1600/in². Even with cap area percentages in a range of 40% to 56%, the peel and shear values of the mechanical fasteners of the present disclosure are better than for a mechanical fastener having a density of mechanical fastening elements of 1600/in². See, for example, a comparison of Examples 1 and 3 vs. Illustrative Examples 2 and 3 and a comparison of Example 7 vs. Illustrative Example 5. On the other hand, with cap area percentages approximately equal at 20% to 30%, mechanical fasteners having a density in a range from 1000/cm² to 5000/cm² have a higher softness and comparable or higher peel and shear performance than mechanical fasteners having a density of fastening elements of 1600/in². See, for example, a comparison of Examples 2 and 4 vs. Illustrative Examples 2 and 3 and a comparison of Example 6 vs. Illustrative Example 5.

### Some Embodiments of the Disclosure

In a first embodiment, the present disclosure provides a mechanical fastener comprising a thermoplastic backing and multiple upstanding fastening elements comprising posts with proximal ends integrally formed with the thermoplastic backing and distal ends comprising spaced-apart caps larger in cross-section area than a cross-sectional area of the posts, wherein the multiple upstanding fastening elements are present in a density in a range from 1000 per square centimeter or 1050 per square centimeter to 5000 per square centimeter, wherein a sum of the cross-sectional area of the spaced-apart caps is in a range from 20 to 70 percent of an area of the mechanical fastener, wherein the spaced-apart caps are circular, oval, or regular polygonal having at least five sides, and wherein the spaced-apart caps do not have a dimension greater than 300 micrometers. In a second embodiment, the present disclosure provides the mechanical fastener of the first embodiment, wherein the spaced-apart caps extend beyond the posts on at least two opposing sides of the posts. In a third embodiment, the present disclosure provides the mechanical fastener of the first or second embodiment, wherein a sum of the cross-sectional area of the caps is in a range from 25% to 70%, 20% to 65%, 20% to 40%, 25% to 60%, or from 31% to 60% of the area of the mechanical fastener. In a fourth embodiment, the present disclosure provides the mechanical fastener of any one of the first to third embodiments, wherein at least a portion of the spaced-apart caps is deflected toward the thermoplastic backing. In a fifth embodiment, the present disclosure provides the mechanical fastener of the any one of the first to fourth embodiments, wherein the density of the multiple upstanding fastening elements is in a range from 1000 per square centimeter to 1400 per square centimeter, and wherein the spaced-apart caps have a dimension in a range from 150 micrometers to 270 micrometers. In a sixth embodiment, the present disclosure provides the mechanical fastener of any one of the first to fifth embodiments, wherein the density of the multiple upstanding fastening elements is in a range from 1600 per square centimeter to 4000 per square centimeter. In a seventh embodiment, the present disclosure provides the mechanical fastener of any one of the first to sixth embodiments, wherein the density of the multiple upstanding fastening elements is in a range from 1500 per square centimeter to 4000 per square centimeter, and wherein the spaced-apart caps have a dimension in a range from 80 micrometers to 245 micrometers.

In an eighth embodiment, the present disclosure provides the mechanical fastener of any one of the first to seventh embodiments, wherein at least a portion of the post decreases in size from the proximal end to the distal end. In a ninth embodiment, the present disclosure provides the mechanical fastener of any one of the first to eighth embodiments, wherein the thermoplastic backing has a thickness in a range from 30 micrometers to 100 micrometers, 30 micrometers to 80 micrometers, 25 micrometers to 80 micrometers, or 35 micrometers to 60 micrometers and/or wherein the mechanical fastener has a bending softness of at least 0.01 (grams of force times centimeter)/centimeter and not more than 0.1, 0.09, 0.08, 0.07, 0.06, or 0.05 (grams of force times centimeter)/centimeter. In a tenth embodiment, the present disclosure provides the mechanical fastener of any one of the first to ninth embodiments, wherein the thermoplastic backing has no stretch-induced molecular orientation. In an eleventh embodiment, the present disclosure provides the mechanical fastener of any one of the first to tenth embodiments, wherein the multiple upstanding fastening elements are substantially evenly spaced. In a twelfth embodiment, the present disclosure provides the mechanical fastener of any one of the first to eleventh embodiments, wherein the multiple upstanding fastening elements have a height in a range from 80 micrometers to 350 micrometers, from 100 micrometers to 300 micrometers, or from 150 micrometers to 300 micrometers. In a thirteenth embodiment, the present disclosure provides the mechanical fastener of any one of the first to twelfth embodiments, wherein the posts have a width dimension "w¹" immediately under the cap in a range from 60 µm to 175 µm. In a fourteenth embodiment, the present disclosure provides the mechanical fastener of any one of the first to thirteenth embodiments, wherein the spaced-apart caps are smooth. In a fifteenth embodiment, the present disclosure provides the mechanical fastener of any one of the first to thirteenth embodiments, wherein the spaced-apart caps are grooved, and wherein the sum of the cross-sectional area of the caps is in a range from 20 to 40 percent of an area of the mechanical fastener. In a sixteenth embodiment, the present disclosure provides the mechanical fastener of any one of the first to fifteenth embodiments, wherein the spaced-apart caps are spaced-apart circular caps. In a seventeenth embodiment, the present disclosure provides the mechanical fastener of any one of the first to sixteenth embodiments having a basis weight in a range from 45 grams per square meter to 95 grams per square meter, from 55 grams per square meter to 95 grams per square meter, or 76 grams per square meter to 95 grams per square meter. In an eighteenth embodiment, the present disclosure provides the mechanical fastener of any one of the first to seventeenth embodiments, wherein the mechanical fastener comprises polypropylene.

In a nineteenth embodiment, the present disclosure provides a laminate comprising the mechanical fastener of any one of the first to eighteenth embodiments attached to a carrier. In a twentieth embodiment, the present disclosure provides the laminate of the nineteenth embodiment, wherein the carrier comprises a nonwoven. In a twenty-first embodiment, the present disclosure provides an absorbent article comprising the mechanical fastener of any one of the first to eighteenth embodiments. In a twenty-second embodiment, the present disclosure provides the absorbent article of the twenty-first embodiment, further comprising a front waist region and a rear waist region, wherein at least one of the front waist region or the rear waist region comprises the mechanical fastener. In a twenty-third embodiment, the present disclosure provides the absorbent article of the twenty-first or twenty-second embodiment, wherein the absorbent article is an open-style diaper. In a twenty-fourth embodiment, the present disclosure provides the absorbent article of the twenty-first embodiment, wherein the absorbent article is a pants-style diaper, and wherein the mechanical fastener is located on a tab on a seam or side panel portion of the pants-style diaper.

In order that this disclosure can be more fully understood, the following examples are set forth. It should be understood that these examples are for illustrative purposes only and are not to be construed as limiting this disclosure in any manner.

### EXAMPLES

### Test Methods

### Peel Evaluation

The mechanical fasteners of Examples 1 to 7 and Illustrative Examples 1 to 5 were evaluated using a nonwoven loop material taken from a "MOONY" diaper from Unicharm, Tokyo, Japan. For peel evaluation, a patch of the mechanical fastener 25 millimeters (mm) in the machine direction (MD) and 20 mm in the cross-direction (CD)) was attached to one end of a paper leader with single-coated tape. The nonwoven material was attached with double sided tape to a metal plate (50 mm by 100 mm (test direction)) with care taken not to scratch or press too strongly on the loop surface. The mechanical fastener specimen was pressed onto the nonwoven using one cycle (one cycle = one forward and one backward pass) with a 700-gram roller with the speed of 300 mm/minute. The materials were oriented so that the peel was conducted in the Example specimen CD and the nonwoven CD. The 135-degree peel was measured using an instrument obtained from A&D Company, Limited, Tokyo, Japan, under the trade designation "TENSILON RTG-1225". The metal plate was fit into the 135-degree peel jig and held stationary, and the edge of the paper tab was clamped. The peel start position was at the vertical bottom of the chuck. A 300 mm per minute separation speed was used. The evaluation was carried out twice, using fresh samples, and the average peel force was recorded. The results are shown in Tables 1 and 2, below.

### Shear Evaluation

The mechanical fasteners of Examples 1 to 7 and Illustrative Examples 1 to 5 were evaluated using a nonwoven loop material taken from a "MOONY" diaper from Unicharm. The finished mechanical fastener test specimen was prepared as a 20 mm CD by 25 mm MD strip attached to a 90 mm by 25 mm leader of filament tape (available from 3M Company, St. Paul, MN). A 70 mm by 25 mm strip of 38-micrometer-thick paper was used to cover the exposed adhesive of the filament tape.

The nonwoven material (50 mm by 100 mm (cross direction)) was attached with double sided tape to a metal plate (50 mm by 100 mm (test direction)) with care taken not to scratch or press too strongly on the loop surface. The mechanical fastener specimen was pressed onto the nonwoven using one cycle (one cycle = one forward and one backward pass) with a 700-gram roller with the speed of 300 mm/minute. The materials were oriented so that the shear was conducted in the Example mechanical fastener CD and the nonwoven CD. The shear was measured using an instrument obtained from A&D Company, Limited, under the trade designation "TENSILON RTG-1225". The leader of the Example specimen was attached to the upper jaw while the loop sample was attached to the lower jaw of the instrument, allowing for a slight amount of slack.

The instrument was started and the upper jaw traveled until the Example specimen was completely disengaged from the loop sample. The instrument crosshead speed was set to 300 mm/minute. The evaluation was carried out twice, using fresh samples. The data was averaged, and the average is reported in Tables 1 and 2.

### Pure Bending Evaluation

Bending softness was measured using a KES-FB2-S Pure Bending Tester from Kato Tech Co., Ltd., Kyoto, Japan. Specimens were prepared for machine direction bending by cutting the mechanical fastener of Examples 1 to 7 and Illustrative Examples 1 to 5 to a strip 20 mm long in the CD and 100 mm long in the MD. Specimens were prepared for cross direction bending by cutting the mechanical fastener of Examples 1 to 7 and Illustrative Examples 1 to 5 to a strip 100 mm long in the CD and 20 mm long in the MD. A specimen was placed in the sample holder and bent in the long dimension both toward the fastening elements side and toward the opposite side. The evaluation was carried out twice, and the data were averaged. The average bending softness is reported in Tables 1 and 2, below.

### Tactile Feel Evaluation

Six people evaluated the tactile feeling of the mechanical fasteners of Examples 1 to 7 and Illustrative Examples 1 to 5 by hand and rated them on a scale of 1 to 10, with 1 being the softest and 10 being the roughest. The average of the six values are provided in Tables 1 and 2 below.

### Basis Weight

Basis weight was determined by weighing a sample of a known area on an analytical balance.

### Measurement of Dimensions

The recorded measurements in Tables 1 and 2, below, were taken at 175 X magnification using a Keyence Corporation Keyence VHX-1000 microscope equipped with a data acquisition software package, a digital range finder, and a monitor.

### Examples (EX) 1 to 5 and Illustrative Examples (IE) 1 to 3

The mechanical fasteners of Examples 1 to 5 and Illustrative Examples 1 to 3 were prepared from polypropylene obtained from Hyosung, Korea, under the trade designation "J842" using the method described in U.S. Patent Nos. 5,845,375 (Miller et al.) except using an extrusion temperature of 255 °C, a mold temperature of 79 °C, and a speed of 30 meters per minutes. The circular holes in the molds for Examples 1 to 4 and Illustrative Example 1 were arranged in staggered arrays with a spacing between two like rows in the X direction (with one staggered row in between) reported as the X pitch and a spacing between directly adjacent staggered rows in the Y direction reported as the Y pitch for a density of holes and resulting upstanding posts shown in Table 1, below. The circular holes in the molds for Illustrative Examples 2 and 3 were arranged in square arrays with a spacing between directly adjacent rows in the X direction reported as the X pitch and a spacing between directly adjacent rows in the Y direction reported as the Y pitch for a density of holes and resulting upstanding posts shown in Table 1, below. The holes were generally conical in shape and had a depth of 350 µm. For Example 5, the thermoplastic backing with upstanding posts was then stretched in the machine direction with a draw ratio of 1.15:1. The upstanding fastening elements were provided with oval, grooved caps according to the process described in U.S. Pat. No. 5,868,987 (Kampfer et al.). The caps were reshaped so that the overhanging portions of the distal cap, that extend beyond the post, were pushed down toward the backing (process for reshaping described in U.S. Pat. No. 6,132,660 (Kampfer)).

In Table 1, the density of the upstanding fastening elements (in number per square inch), the X pitch, the Y pitch, the basis weight, the width of the cap in the cross direction (CD) and machine direction (MD), the post widths "w" adjacent the thermoplastic backing and "w1" adjacent the cap, and the backing thickness are recorded in Table 1. The cap area percent was calculated from the CD cap width and MD cap width.

Examples 1 to 5 and Illustrative Examples 1 to 3 were evaluated for tactile smoothness, KES softness in the MD and CD, peel, and shear, using the test methods described above. The results are provided in Table 1, below.

**Table 1. Examples (EX) 1 to 5, and Illustrative Examples (IE) 1 to 3 with Oval, Grooved Caps**

| | EX1 | EX2 | EX3 | EX4 | EX5 | IE1 | IE2 | IE3 |
|---|---|---|---|---|---|---|---|---|
| Density, #/in.² (#/cm²) | 8000 (1240) | 8000 (1240) | 8000 (1240) | 8000 (1240) | 7000 (1085) | 3500 (543) | 1600 (248) | 1600 (248) |
| X Pitch, µm | 481 | 481 | 481 | 481 | 513 | 778 | 588 | 580 |
| Y Pitch, µm | 141 | 141 | 141 | 141 | 143 | 201 | 596 | 593 |
| Basis weight, gsm | 83 | 83 | 83 | 83 | 75 | 60 | 65 | 100 |
| CD cap width, µm | 251 | 185 | 209 | 148 | 176 | 304 | 379 | 404 |
| MD cap width, µm | 153 | 136 | 159 | 127 | 139 | 178 | 240 | 292 |
| Height, µm | 294 | 325 | 289 | 325 | 304 | 133 | 187 | 297 |
| Backing thickness, µm | 45 | 44 | 53 | 45 | 38 | 51 | 59 | 80 |
| Post width base, µm | 183 | 176 | 173 | 182 | 166 | 164 | 185 | 280 |
| Post width neck, µm | 145 | 122 | 122 | 117 | 120 | 167 | 179 | 208 |
| Cap area percent | 44% | 29% | 39% | 22% | 26% | 27% | 20% | 27% |
| Tactile Smoothness | 6.8 | 5.2 | 5.0 | 3.8 | 6.2 | 7.0 | 10.0 | 7.2 |
| KES softness MD, gf*cm/cm | 0.0545 | 0.053 | 0.044 | 0.049 | 0.021 | 0.057 | 0.070 | 0.205 |
| KES softness CD, gf*cm/cm | 0.0455 | 0.044 | 0.049 | 0.041 | 0.024 | 0.058 | 0.067 | 0.160 |
| Peel, N/25mm | 0.59 | 0.66 | 0.74 | 0.42 | 0.58 | 0.51 | 0.35 | 0.35 |
| Shear, N/25mm | 40.0 | 44.5 | 46.5 | 27.0 | 50.5 | 43.5 | 30.5 | 30.4 |

### Examples (EX) 6 and 7 and Illustrative Examples (IE) 4 and 5

The mechanical fasteners of Example 7 and Illustrative Examples 4 and 5 were prepared as described for Examples 1 to 5 and Illustrative Examples 1 to 3. For Example 7 and Illustrative Example 4, a staggered array was used, and for Illustrative Example 5, a square array was used, both with the X and Y pitch defined as described above. The mechanical fastener of Example 6 was prepared as described for Examples 1 to 5 and Illustrative Examples 1 to 3 with the modification that polypropylene obtained from Braskem, Sao Paulo, Brazil, under the trade designation "C700-35N" was used for Example 6, and the mold had regular hexagonal holes in a staggered array with the X and Y pitch defined as described above.

For Examples 6 and 7 and Illustrative Examples 4 and 5, instead of using the process described in U.S. Pat. No. 5,868,987 (Kampfer et al.), a flat capping roller was used to form smooth caps. The caps of Example 7 and Illustrative Examples 4 and 5 were generally circular. The caps of Example 6 were generally hexagonal with radiused angles. Then, the caps were reshaped so that the overhanging portions of the distal cap, that extend beyond the post, were pushed down toward the backing (process for reshaping described in U.S. Pat. No. 6,132,660 (Kampfer)).

In Table 2, the density of the upstanding fastening elements (in number per square inch), the X pitch, the Y pitch, the basis weight, the width of the cap in the cross direction (CD) and machine direction (MD), the post widths "w" adjacent the thermoplastic backing and "w¹" adjacent the cap, and the backing thickness are recorded in Table 2. The cap area percent was calculated from the CD cap width and MD cap width.

Examples 6 and 7 and Illustrative Examples 4 and 5 were evaluated for tactile smoothness, KES softness in the MD and CD, peel, and shear, using the test methods described above. The results are provided in Table 2, below.

**Table 2. Examples 6 and 7 and Illustrative Examples 4 and 5 with Smooth Caps**

| | EX6 | EX7 | IE4 | IE5 |
|---|---|---|---|---|
| Density, #/in.² (#/cm²) | 16000 (2481) | 8000 (1240) | 3500 (543) | 1600 (248) |
| X Pitch, µm | 323 | 481 | 778 | 588 |
| Y Pitch, µm | 97 | 141 | 201 | 596 |
| Basis weight, gsm | 90 | 73 | 60 | 65 |
| CD cap width, µm | 104 | 208 | 258 | 353 |
| MD cap width, µm | 107 | 233 | 237 | 352 |
| Height, µm | 107 | 227 | 122 | 158 |
| Backing thickness, µm | 81 | 47 | 51 | 64 |
| Post width base, µm | 135 | 175 | 150 | 188 |
| Post width neck, µm | 81 | 132 | 150 | 186 |
| Cap area percent | 28% | 56% | 31% | 28% |
| Tactile Smoothness | 1.5 | 1.5 | 3.3 | 8.7 |
| KES softness MD, gf*cm/cm | 0.082 | 0.022 | 0.055 | 0.178 |
| KES softness CD, gf*cm/cm | 0.073 | 0.022 | 0.059 | 0.170 |
| Peel, N/25mm | 0.27 | 0.41 | 0.47 | 0.30 |
| Shear, N/25mm | 48.5 | 28.0 | 31.0 | 26.5 |

This disclosure is not limited to the above-described embodiments but is to be controlled by the limitations set forth in the following claims.

## Claims

1. A mechanical fastener comprising:
a thermoplastic backing; and
multiple upstanding fastening elements comprising posts with proximal ends integrally formed with the thermoplastic backing and distal ends comprising spaced-apart caps larger in cross-section area than a cross-sectional area of the posts,
wherein the multiple upstanding fastening elements are present in a density in a range from 1000 per square centimeter to 5000 per square centimeter, wherein a sum of the cross-sectional area of the spaced-apart caps is in a range from 20 to 70 percent of an area of the mechanical fastener, wherein the spaced-apart caps are circular, oval, or regular polygonal having at least five sides, and wherein the spaced-apart caps do not have a dimension greater than 300 micrometers.

2. The mechanical fastener of claim 1, wherein the spaced-apart caps extend beyond the posts on at least two opposing sides of the posts.

3. The mechanical fastener of claim 1 or 2, wherein at least a portion of the spaced-apart caps is deflected toward the thermoplastic backing.

4. The mechanical fastener of any one of claims 1 to 3, wherein the density of the multiple upstanding fastening elements is in a range from 1000 per square centimeter to 1400 per square centimeter, and wherein the spaced-apart caps have a dimension in a range from 150 micrometers to 270 micrometers.

5. The mechanical fastener of any one of claims 1 to 3, wherein the density of the multiple upstanding fastening elements is in a range from 1600 per square centimeter to 4000 per square centimeter.

6. The mechanical fastener of any one of claims 1 to 3, wherein the density of the multiple upstanding fastening elements is in a range from 1500 per square centimeter to 4000 per square centimeter, and wherein the spaced-apart caps have a dimension in a range from 80 micrometers to 245 micrometers.

7. The mechanical fastener of any one of claims 1 to 6, wherein at least one of the following limitations is met:
the thermoplastic backing has a thickness in a range from 25 micrometers to 80 micrometers; or
the mechanical fastener has a bending softness of at least 0.01 (grams of force times centimeter)/centimeter and not more than 0.1 (grams of force times centimeter)/centimeter.

8. The mechanical fastener of any one of claims 1 to 7, wherein the thermoplastic backing has no stretch-induced molecular orientation.

9. The mechanical fastener of any one of claims 1 to 8, wherein the multiple upstanding fastening elements are substantially evenly spaced.

10. The mechanical fastener of any one of claims 1 to 9, wherein the multiple upstanding fastening elements have a height in a range from 100 micrometers to 300 micrometers.

11. The mechanical fastener of any one of claims 1 to 10, wherein the spaced-apart caps are smooth.

12. The mechanical fastener of any one of claims 1 to 11, wherein the spaced-apart caps are spaced-apart circular caps.

13. The mechanical fastener of any one of claims 1 to 12 having a basis weight in a range from 45 grams per square meter to 95 grams per square meter or 76 grams per square meter to 95 grams per square meter.

14. A laminate comprising the mechanical fastener of any one of claims 1 to 13 attached to a carrier.

15. An absorbent article comprising the mechanical fastener of any one of claims 1 to 13.

## Patentansprüche

1. Ein mechanisches Befestigungsmittel, aufweisend:
eine thermoplastische Rückseite; und
vielfache aufrechte Befestigungselemente, aufweisend Stifte mit proximalen Enden, die mit der thermoplastischen Rückseite einstückig ausgebildet sind, und distale Enden, aufweisend beabstandete Kappen, die in der Querschnittsfläche größer als eine Querschnittsfläche der Stifte sind,
wobei die vielfachen aufrechten Befestigungselemente in einer Dichte in einem Bereich von 1000 pro Quadratzentimeter bis 5000 pro Quadratzentimeter vorhanden sind, wobei eine Summe der Querschnittsfläche der beabstandeten Kappen in einem Bereich von 20 bis 70 Prozent einer Fläche des mechanischen Befestigungsmittels liegt, wobei die beabstandeten Kappen kreisförmig, oval oder regelmäßig polygonal, die mindestens fünf Seiten vorweisen, sind, und wobei die beabstandeten Kappen keine Abmessung größer als 300 Mikrometer vorweisen.

2. Das mechanische Befestigungsmittel nach Anspruch 1, wobei sich die beabstandeten Kappen über die Stifte hinaus an mindestens zwei gegenüberliegenden Seiten der Stifte erstrecken.

3. Das mechanische Befestigungsmittel nach Anspruch 1 oder 2, wobei mindestens ein Abschnitt der beabstandeten Kappen zu der thermoplastischen Rückseite hin abgelenkt ist.

4. Das mechanische Befestigungsmittel nach einem der Ansprüche 1 bis 3, wobei die Dichte der vielfachen aufrechten Befestigungselemente in einem Bereich von 1000 pro Quadratzentimeter bis 1400 pro Quadratzentimeter liegt, und wobei die beabstandeten Kappen eine Abmessung in einem Bereich von 150 Mikrometer bis 270 Mikrometer vorweisen.

5. Das mechanische Befestigungsmittel nach einem der Ansprüche 1 bis 3, wobei die Dichte der vielfachen aufrechten Befestigungselemente in einem Bereich von 1600 pro Quadratzentimeter bis 4000 pro Quadratzentimeter liegt.

6. Das mechanische Befestigungsmittel nach einem der Ansprüche 1 bis 3, wobei die Dichte der vielfachen aufrechten Befestigungselemente in einem Bereich von 1500 pro Quadratzentimeter bis 4000 pro Quadratzentimeter liegt, und wobei die beabstandeten Kappen eine Abmessung in einem Bereich von 80 Mikrometer bis 245 Mikrometer vorweisen.

7. Das mechanische Befestigungsmittel nach einem der Ansprüche 1 bis 6, wobei mindestens eine der folgenden Einschränkungen erfüllt ist:
die thermoplastische Rückseite eine Dicke in einem Bereich von 25 Mikrometer bis 80 Mikrometer aufweist; oder
das mechanische Befestigungsmittel eine Biegeweichheit von mindestens 0,01 (Gramm Kraft mal Zentimeter)/Zentimeter und nicht mehr als 0,1 (Gramm Kraft mal Zentimeter)/Zentimeter vorweist.

8. Das mechanische Befestigungsmittel nach einem der Ansprüche 1 bis 7, wobei die thermoplastische Rückseite keine durch Dehnung hervorgerufene molekulare Ausrichtung vorweist.

9. Das mechanische Befestigungsmittel nach einem der Ansprüche 1 bis 8, wobei die vielfachen aufrechten Befestigungselemente im Wesentlichen gleichmäßig beabstandet sind.

10. Das mechanische Befestigungsmittel nach einem der Ansprüche 1 bis 9, wobei die vielfachen aufrechten Befestigungselemente eine Höhe in dem Bereich von 100 Mikrometer bis 300 Mikrometer vorweisen.

11. Das mechanische Befestigungsmittel nach einem der Ansprüche 1 bis 10, wobei beabstandeten Kappen glatt sind.

12. Das mechanische Befestigungsmittel nach einem der Ansprüche 1 bis 11, wobei die beabstandeten Kappen beabstandete kreisförmige Kappen sind.

13. Das mechanische Befestigungsmittel nach einem der Ansprüche 1 bis 12, das ein Flächengewicht in einem Bereich von 45 Gramm pro Quadratmeter bis 95 Gramm pro Quadratmeter oder 76 Gramm pro Quadratmeter bis 95 Gramm pro Quadratmeter vorweist.

14. Ein Laminat, aufweisend das mechanische Befestigungsmittel nach einem der Ansprüche 1 bis 13, das an einen Träger angebracht ist.

15. Ein Absorptionsartikel, aufweisend das mechanische Befestigungsmittel nach einem der Ansprüche 1 bis 13.

## Revendications

1. Élément de fixation mécanique comprenant :
un support thermoplastique ; et
plusieurs éléments de fixation verticaux comprenant des montants avec des extrémités proximales formées intégralement avec le support thermoplastique et des extrémités distales comprenant des capuchons espacés plus grands en surface de section transversale qu'une aire en coupe transversale des montants,
dans lequel les multiples éléments de fixation verticaux sont présents dans une densité comprise entre 1000 par centimètre carré et 5000 par centimètre carré, dans lequel une somme de l'aire de section transversale des capuchons espacés est comprise dans une plage allant de 20 à 70 pour cent d'une aire de la fixation mécanique, dans laquelle les capuchons espacés sont circulaires, ovale ou polygonal régulier ayant au moins cinq côtés, et dans lequel les capuchons espacés n'ont pas une dimension supérieure à 300 micromètres.

2. Élément de fixation mécanique selon la revendication 1, dans lequel les capuchons espacés s'étendent au-delà des montants sur au moins deux côtés opposés des montants.

3. Élément de fixation mécanique selon la revendication 1 ou 2, dans lequel au moins une partie des capuchons espacés est déviée vers le support thermoplastique.

4. Élément de fixation mécanique selon l'une quelconque des revendications 1 à 3, dans lequel la densité des multiples éléments de fixation verticaux est dans une plage allant de 1000 par centimètre carré à 1400 par centimètre carré, et dans lequel les capuchons espacés ont une dimension dans une plage allant de 150 micromètres à 270 micromètres.

5. Élément de fixation mécanique selon l'une quelconque des revendications 1 à 3, dans lequel la densité des multiples éléments de fixation verticaux est dans une plage allant de 1600 par centimètre carré à 4000 par centimètre carré.

6. Élément de fixation mécanique selon l'une quelconque des revendications 1 à 3, dans lequel la densité des multiples éléments de fixation verticaux est dans une plage allant de 1500 par centimètre carré à 4000 par centimètre carré, et dans lequel les capuchons espacés ont une dimension dans une plage allant de 80 micromètres à 245 micromètres.

7. Élément de fixation mécanique selon l'une quelconque des revendications 1 à 6, dans lequel au moins l'une des conditions suivantes est satisfaite :
le support thermoplastique a une épaisseur dans une plage allant de 25 micromètres à 80 micromètres ; ou
l'élément de fixation mécanique a une souplesse de flexion d'au moins 0,01 (grammes de force multipliés par centimètre)/centimètre et ne dépassant pas 0,1 (grammes de force multipliés par centimètre)/centimètre.

8. Élément de fixation mécanique selon l'une quelconque des revendications 1 à 7, dans lequel le support thermoplastique n'a pas d'orientation moléculaire induite par étirement.

9. Élément de fixation mécanique selon l'une quelconque des revendications 1 à 8, dans lequel les multiples éléments de fixation verticaux sont sensiblement régulièrement espacés.

10. Élément de fixation mécanique selon l'une quelconque des revendications 1 à 9, dans lequel les multiples éléments de fixation verticaux ont une hauteur dans la plage allant de 100 micromètre à 300 micromètres.

11. Élément de fixation mécanique selon l'une quelconque des revendications 1 à 10, dans lequel les capuchons espacés sont lisses.

12. Élément de fixation mécanique selon l'une quelconque des revendications 1 à 11, dans lequel les capuchons espacés sont des capuchons circulaires espacés.

13. Élément de fixation mécanique selon l'une quelconque des revendications 1 à 12, ayant un poids de base dans une plage allant de 45 grammes par mètre carré à 95 grammes par mètre carré ou de 76 grammes par mètre carré à 95 grammes par mètre carré.

14. Stratifié comprenant l'élément de fixation mécanique selon l'une quelconque des revendications 1 à 13, attaché à un support.

15. Article absorbant comprenant l'élément de fixation mécanique selon l'une quelconque des revendications 1 à 13.
